# EUROPÄISCHE PATENTSCHRIFT

(11) **EP 3 340 958 B1**
(45) Veröffentlichungstag und Bekanntmachung des Hinweises auf die Patenterteilung: **14.10.2020**
(21) Anmeldenummer: 15820445.3
(22) Anmeldetag: 07.12.2015
(51) Int. Cl.: A61G 13/10

(54) **VORRICHTUNG ZUM BEFESTIGEN EINES ZUBEHÖRTEILS AN EINEM OPERATIONSTISCH**
DEVICE FOR SECURING AN ACCESSORY TO AN OPERATING TABLE
DISPOSITIF POUR FIXER UN ACCESSOIRE À UNE TABLE D'OPÉRATION

(30) Priorität: 28.08.2015 DE 102015114402
(43) Veröffentlichungstag der Anmeldung: 04.07.2018
(73) Patentinhaber: Maquet GmbH, 76437 Rastatt (DE)
(72) Erfinder: KATZENSTEIN, Bernhard, 76473 Iffezheim (DE)
(74) Vertreter: Zacco GmbH
(86) Internationale Anmeldenummer: PCT/EP2015/078789
(87) Internationale Veröffentlichungsnummer: WO 2017/036567

(56) Entgegenhaltungen:
- JP-U- H0 316 926
- US-A- 5 836 559
- US-B1- 8 051 515
- US-B1- 9 022 334

## Beschreibung

Die Erfindung betrifft eine Vorrichtung zum Befestigen eines Zubehörteils an einem Operationstisch, die einen Grundkörper, eine erste Befestigungseinheit zur Befestigung der Vorrichtung an dem Operationstisch, insbesondere an einer Schiene des Operationstisches, und eine zweite Befestigungseinheit zur Befestigung einer Stange des Zubehörteils an der Vorrichtung umfasst.

Die Patienten müssen bei Operationen je nach Eingriff in verschiedenen Positionen gelagert werden. Dies kann z.B. bei einer Operation an den Nieren eine Seitenlage sein. Da der Patient in solchen Lagen nicht stabil liegt, benötigt man eine Vorrichtung, um den Patienten in einer Lage zu stützen bzw. zu stabilisieren.

Hierfür werden sogenannte Körperstützen verwendet, um einen Patienten in einer vorbestimmten Lage auf dem Operationstisch zu stabiliseren. Die Körperstützen werden an an den Seiten des Operstionstisches befindlichen Schienen befestigt. Um die körperlich unterschiedlich ausgebildeten Patienten optimal zu stützen, müssen die Körperstützen in der Höhe verstellbar und in eingestellter Höhe fixierbar sein. Auf diese Körperstützen wirken also Kräfte von außen ein, welche über die Befestigungseinrichtungen in den Operationstisch eingeleitet werden. Solche Vorrichtungen zur Befestigung der Zubehörteile werden häufig auch als Kloben bezeichnet.

Bei den einzuleitenden Kräften handelt es sich um Momente, welche um eine Achse parallel zum Operationstisch eingeleitet werden. Da die Schienen nicht entlang der kompletten Lagerfläche ausgebildet sind, benötigt man gegebenenfalls auch Stützen, welche den Patienten an den Stellen, an denen direkt unterhalb keine Schiene angeordnet ist, stützen können. In diesen Fällen müssen auch Momente um eine Achse, welche senkrecht zur Oberfläche des Operationstisches steht, mit Hilfe der Kloben in die Schienen eingeleitet werden.

Bei den einzuleitenden Kräften handelt es sich auch um solche, welche den Patienten so abstützen, dass dieser bei einer zur horizontalen Ebene geneigten Einstellung der Lagerfläche, z.B. bei einer Trendelenburg Lagerung, nicht von der Lagerfläche herunterrutschen kann. Hierfür werden üblicher Weise sogenannte Schulterstützen oder Fußstützen verwendet, welche ebenfalls über einen Kloben mit der seitlich angebrachten Gleitschiene des Operationstisches verbunden sind. Die Momente die hier gestützt werden müssen sind also Momente um eine Achse, die parallel zu Oberfläche der Lagerfläche und rechtwinklig zu Ihrer Längsausrichtung angeordnet ist.

Da einerseits die Orientierung der Lagerfläche im Raum auch durch das gleichzeitige Neigen in Längsrichtung und um eine Achse, die parallel zur Lagerflächenlängsachse angeordnet ist, möglich ist, und andererseits auch Zubehörteile, wie Instrumentenablagen, Infusionsständer und Narkosebögen, also Zubehörteile, die den Pateinten nicht abstützen, aber die für ein angenehmes Arbeiten oder auch für die Befestigung von Abdeckmaterial, Schläuchen, Leitungen oder Geräten zur intraoperativen Medikamentenabgabe benötigt werden, über die Kloben befestigt werden, sind beliebige Kombinationen von Momenten und Kräften auf die Kloben möglich, welche sicher gehalten werden müssen.

Eine Möglichkeit, um eine möglichst sichere Einleitung der Kräfte und Momente über die Kloben zu gewährleisten, ist es, die Körperstützen stoffschlüssig mit den Kloben zu verbinden. Dies hat den Nachteil, dass der Kloben fest mit dem Zubehör verbunden ist, wodurch sich eine hohe Gesamtmasse des Zubehörs ergibt. Ferner ist es nachteilig, dass für jedes Zubehörteil auch ein Kloben an diesem befestigt sein muss, was die Kosten und den benötigten Lagerraum erhöht.

Ferner sind Systeme bekannt, bei denen die Körperstützen formschlüssig über spezielle Kloben befestigt werden. Bei den bekannten Systemen ist immer nur ein Kloben für eine bestimmte Körperstütze oder eine Körperstützenserie geeignet. Die Stangen der Körperstützen weisen häufig rechteckige, quadratische oder runde Profile auf. Auch sind sechs- oder achtkantige Profile bekannt. Bei den bekannten Kloben muß für jede Profilart ein eigener Kloben verwendet werden, was den Nachteil hat, dass eine Vielzahl verschiedenener Kloben bevorratet werden muss und es leicht dazu kommt, dass zunächst versucht wird, den falschen Kloben zu verwenden.

Ferner werden über solche Kloben auch beispielsweise Narkosebögen an den Schienen des Operationstisches befestigt. Ein Narkosebogen ist ein Gestänge, welches seitlich an den Gleitschienen einer Lagerfläche im Bereich zwischen Patientenhals und Patientenbrust angebracht wird. An diesen Gestängen werden zum einen die sterilen Tücher befestigt, welche den Patienten abdecken, um den Anästhesisten einen möglichst guten Zugang zum Kopf des Patienten zu ermöglichen. Zum anderen können an den Gestängen die Arme des Patienten fixiert werden, um dem chirurgischen Team den Zutritt zum Operationsfeld zu vereinfachen. Auch bei den Narkosebögen sind viele verschiedenen Profile bekannt, wobei auch hier eigene Kloben für jede Profilart verwendet werden.

Darüber hinaus werden über Kloben ebenfalls Armlagerungen für die interoperative Lagerung des Patientenarms an den Schienen befestigt.

Darüber hinaus sind sogenannte Radialstellkloben zum Verbinden von Zubehörstangen mit Operationstischen bekannt. Diese sind dadurch gekennzeichnet, daß die Zubehörstangen zusätzlich um eine Achse, welche einerseits in einer Ebene parallel zur Oberfläche eines Operationsisches und andererseits rechtwinklig zur Längsausrichtung des Operationstisches ausgerichtet ist, rotiert und winklig zur Oberfläche des Operationstisches fixiert werden kann. Die Fixierung und die winklige Feststellung der Stangen erfolgt mit einem manuell bedienbaren Betätigungselement, wobei hierfür häufig eine Knebelschraube als manuell zu betätigendes Befestigungselement verwendung findet. Solche Radialstellkloben sind jedoch ausschließlich auf die Aufnahme von Stangen mit runden Profilen ausgelegt. Zubehörteile mit Stangen mit rechteckigem Profil können durch diese Radialstellkloben nicht an den Schienen befestigt werden. Desweiteren besteht hier der Nachteil, dass mit dem Lösen der Verspannung durch die Knebelschraube alle Freiheitsgrade gleichzeitig gelöst werden, so dass ein feinfühliges Einstellen der Position der über den Radialstellkloben befestigten Zubehörteile nicht möglich ist.

Das Dokument JP H03 16926 U beschreibt eine herkömmliche Vorrichtung zum Befestigen von Zubehörteilen an einem Operationstisch.

Es ist Aufgabe der Erfindung, eine Vorrichtung zum Befestigen von Zubehörteilen an einem Operationstisch anzugeben, mit deren Hilfe eine große Anzahl verschiedener Zubehörteile unabhängig von deren Profil an den Operationstisch einfach und sicher befestigt werden kann.

Diese Aufgabe wird duch eine Vorrichtung mit den Merkmalen des Anspruchs 1 gelöst. Vorteilhafte Weiterbildungen der Erfindungen sind in den abhängigen Ansprüchen angegeben.

Erfindungsgemäß weist die zweite Befestigungseinheit ein Loch zur Aufnahme einer Stange des Zubehörteils sowie eine Klemmeinheit zum Einklemmen einer im Loch aufgenommenen Stange auf. Der Querschnitt des Loches ist hierbei derart ausgebildet, dass in dem Loch sowohl Stangen mit einer ersten Profilart als auch Stangen mit mindestens einer zweiten Profilart mit Hilfe der Klemmeinheit befestigbar sind. Insbesondere sind sowohl Stangen der ersten als auch der zweiten Profilart formschlüssig in dem Loch aufnehmbar. Der Querschnitt des Loches ergibt sich aus der Überlagerung eines Quadrates und eines oder zweier Rechtecke. Ferner ist die Diagonale des Quadrates länger als die längere Seite eines jeden Rechtecks.

Mit der zuvor beschriebenen Vorrichtung wird erreicht, dass zumindest Stangen mit dem ersten und dem zweiten Profil mit Hilfe derselben Vorrichtung an einem Operationstisch befestigt werden können. Dabei muss, anders als bei bekanntem Stand der Technik, nicht für jede Profilform eine eigene Vorrichtung verwendet werden, deren Aufnahmebereich an die entsprechende Profilform angepasst ist. Somit werden Verwechslungen vorgebeugt und die Handhabung vereinfacht. Ferner müssen nicht mehr so viele Teile bevorratet werden, was die Kosten und den notwendigen Lagerraum minimiert.

Unter dem Profil wird im Rahmen dieser Anmeldung insbesondere die äußere Form des Querschnitts der jeweiligen Stange verstanden. Die Stangen können somit insbesondere ein rechteckiges, ein rundes oder ein quadratisches Profil haben, wobei im Rahmen dieser Anmeldung unter einem rechteckigen Profil insbesondere verstanden wird, dass dieses Profil nicht quadratisch ist.

Das Loch ist hierbei insbesondere derart ausgebildet, dass die Formgebung seiner Wandung sowohl auf die erste als auch auf die zweite Profilart abgestimmt ist.

Unter Profilart wird die jeweilige Art des Querschnitts verstanden, also beispielsweise Rundstangen, rechteckige Stangen etc. Unter unterschiedlichen Profilarten werden nicht Stangen mit gleicher Form jedoch unterscheidlicher Abmessung verstanden, beispielsweise nicht zwei Rundprofile mit unterschiedlichen Durchmessern.

Das Loch ist insbesondere derart geformt, dass es in einem Teilbereich komplementär zu der ersten Profilart und in einem zweiten Teilbereich komplementär zu der zweiten Profilart ausgebildet ist. Hierdurch wird erreicht, dass sowohl Stangen mit der ersten Profilart als auch Stangen mit der zweiten Profilart sicher eingeklemmt werden können, da sie die Wandung des Loches über einen möglichst großen Bereich kontaktieren.

In einer besonders bevorzugten Ausführungsform ist der Querschnitt des Loches derart ausgebildet, dass in dem Loch auch Stangen mit mindestens einer dritten Profilart befestigbar sind. Hierdurch wird erreicht, dass noch eine größere Anzahl von unterschiedlichen Profilen aufgenommen werden kann, so dass mit Hilfe nur einer Vorrichtung möglichst alle gängigen Profilarten an dem Operationstisch befestigt werden können.

Insbesondere ist das Loch derart ausgebildet, dass Stangen mit einem kreisrunden Profil, Stangen mit einem kreisrunden Profil mit planen Flächen, Stangen mit einem rechteckigen Profil und/oder Stangen mit einem n-eckigen, achsensymmetrischen Profil jeweils innerhalb eines vorbestimmten Abmessungsbereichs aufnehmbar sind. Hierdurch wird erreicht, dass alle gängigen Profile innerhalb der üblichen Abmessungsbereiche über die Vorrichtung an dem Operationstisch befestigt werden können. Unter einem n-eckigen achsensymmetrischen Profil wird beispielsweise ein symmetrisches sechseckiges oder symmetrisches achteckiges Profil verstanden.

Ferner ist es vorteilhaft, wenn der Querschnitt des Loches derart ausgebildet ist, dass sowohl Stangen der ersten Profilart als auch Stangen der zweiten Profilart das Loch an der der Klemmeinheit entgegengesetzten Seite entlang mindestens dreier Punktkontakte oder zweier Linien kontaktieren. Diese beiden Linien liegen insbesondere nicht auf einer gemeinsamen Achse sondern sind parallel zueinander angeordnet. Über den Kontakt entlang zweier Linien oder dreier Punktkontakte wird eine sichere Befestigung sowohl von Stangen der ersten Profilart als auch von Stangen der zweiten Profilart in dem Loch sichergestellt. Insbesondere wird über den Kontakt an mehr als einer Linie ein Verdrehen oder Verkippen der jeweiligen Stange sicher vermieden.

Es ist besonders vorteilhaft, wenn der Querschnitt des Loches derart ausgebildet ist, dass zumindest Stangen der ersten Profilart das Loch an der der Klemmeinheit entgegengesetzten Seite flächig kontaktieren. Über den flächigen Kontakt wird ein besonders sicherer Halt gewährleistet. Insbesondere können so auch größere Kräfte ohne Beschädigungen der Vorrichtung und/oder der Stangen, insbesondere bei Hohlprofilen, sichergestellt werden. Es ist besonders vorteilhaft, wenn auch Stangen der zweiten Profilart, wenn sie in dem Loch aufgenommen sind, die Wandung, die der Klemmeinheit entgegengesetzt ist, flächig kontaktieren.

Bei einer besonders bevorzugten Ausführungsform der Erfindung ist der Querschnitt des Loches derart ausgebildet, dass Stangen der zweiten Profilart derart in dem Loch eingespannt werden, dass ein seitliches Verrutschen der aufgenommenen Stangen über den Kontakt zur Wandung des Loches vermieden wird. Hierzu ist das Loch insbesondere derart geformt, dass die Stangen unabhängig von ihrer Profilart an mindestens zwei Seiten kontaktiert werden, so dass ein Verrutschen und Verdrehen verhindert wird.

Bei einer besonders bevorzugten Ausführungsform der Erfindung ergibt sich der Querschnitt des Loches aus der Überlagerung eines Quadrates und eines Rechtecks, wobei das Quadrat insbesondere relativ zum Rechteck um 45° gedreht ist. Hierunter wird insbesondere verstanden, dass die Mittelpunkte zusammenfallen und die Diagonalen um 45° zueinander gedreht sind.

Durch diese Formgebung wird erreicht, dass sowohl rechteckige, quadratische, runde als auch n-eckige, achsensymmetrische Profile sicher eingeklemmt werden können, da sie in einer der Klemmeinheit entgegengesetzten Seite die Wandung des Loches jeweils an mehreren Stellen oder flächig derart kontaktieren, dass ein Verrutschen, Verdrehen und/oder Verkippen der Stangen sicher vermieden wird.

Es ist besonders vorteilhaft, wenn der Querschnitt des Loches teilweise von dem Quadrat und teilweise von dem Rechteck gebildet wird. Die Diagonale des Quadrates ist länger als die längste Seite des Rechtecks, so dass auch an den kurzen Seiten des Rechtecks über die überstehenden Ecken des Quadrates eine V-förmige Nut gebildet wird. Durch diese wird erreicht, dass insbesondere quadratische Profile dreh- und verkippsicher eingespannt werden können, indem eine Kante des quadratischen Profils in der entsprechend gegenüberliegenden V-förmigen Nut angeordnet ist.

Bei einer alternativen Ausführungsform der Erfindung kann der Querschnitt des Loches sich auch aus der Überlagerung eines Quadrates und zweier Rechtecke ergeben, wobei die Rechtecke insbesondere zueinander um 90° und das Quadrat relativ zu den Rechtecken jeweils um 45° gedreht angeordnet sind. Die Rechtecke sind insbesondere derart angeordnet, dass die Mittelpunkte zusammenfallen und die längeren Seiten der beiden Rechtecke zueinander orthogonal angeordnet sind.

Durch diese Formgebung des Querschnitts wird die Anzahl der möglichen Profile, die über die Vorrichtung sicher an dem Operationstisch befestigt werden können noch weiter erhöht. Insbesondere können somit rechteckige Profile in zwei verschiedenen um 90° gedrehten Ausrichtungen aufgenommen werden.

Das Quadrat und die Rechtecke sind insbesondere derart angeordnet, dass sie jeweils übereinander überstehen, so dass der Querschnitt des Loches teilweise von dem Quadrat, teilweise von dem einen Rechteck und teilweise von dem anderen Rechteck begrenzt wird.

Die Rechtecke sind vorzugsweise identisch geformt, wobei die Diagonale des Quadrates länger ist als die längeren Seiten der Rechtecke. Hierdurch wird wiederum erreicht, dass in den kurzen Seiten der Rechtecke V-förmige Nuten zur Aufnahme der Kanten von quadratischen Profilen vorgesehen sind.

Ferner ist vorteilhaft, wenn die Klemmeinheit als eine Spannschraube, insbesondere eine Knebelschraube ausgebildet ist. Somit kann auf einfache Weise ein Klemmen und auch wieder ein Lösen der Befestigung der Stange erreicht werden. Die Spannschraube weist insbesondere eine ebene Kontaktfläche zum Kontaktieren der einzuklemmenden Stangen auf. Hierdurch wird erreicht, dass sowohl runde, rechteckige als auch quadratische Stangen sicher eingeklemmt werden, indem ein Kontakt entlang einer Linie und/oder sogar einer Fläche gegeben ist.

Die erste Befestigungseinheit der Vorrichtung ist vorzugsweise als eine Klemmeinheit zum Festklemmen der Vorrichtung auf der Schiene eines Operationstisches ausgebildet. Die Klemmeinheit weist insbesondere einen ersten Haken auf, über den die Vorrichtung an der oberen Kante der Schiene eingehangen werden kann. Ferner ist ein zweiter Haken vorgesehen, der mit Hilfe eines manuell betätigbaren Hebels bewegt werden kann und der von unten um die Schiene greifen kann. Somit wird ein sicheres Festklemmen in einer gewünschten Position ermöglicht. Hierdurch wird ein Herunterfallen der Vorrichtung von der Schiene und/oder ein seitliches, ungewolltes Verschieben verhindert.

Weitere Merkmale und Vorteile der Erfindung ergeben sich aus der folgenden Beschreibung, die die Erfindung anhand von Ausführungsbeispielen zusammen mit den beigefügten Figuren näher erläutert.

Es zeigen:
- Figur 1: eine schematische, perspektivische Darstellung einer Vorrichtung zum Befestigen eines Zubehörteils an einem Operationstisch;
- Figur 2: eine weitere schematische, perspektivische Darstellung der Vorrichtung nach Figur 1;
- Figur 3: eine weitere schematische, perspektivische Darstellung der Vorrichtung nach Figur 1;
- Figur 4: eine weitere schematische, perspektivische Darstellung der Vorrichtung nach Figur 1;
- Figur 5: eine Draufsicht auf die Vorrichtung nach den Figuren 1 bis 4 bei Aufnahme einer Stange mit einem runden Profil;
- Figur 6: eine Draufsicht auf die Vorrichtung nach den Figuren 1 bis 4 bei Aufnahme einer Stange mit einem rechteckigen Profil;
- Figur 7: eine Draufsicht auf die Vorrichtung nach den Figuren 1 bis 4 bei Aufnahme einer weiteren rechteckigen Stange;
- Figur 8: eine Draufsicht auf die Vorrichtung nach den Figuren 1 bis 4 bei Aufnahme einer Stange mit einem quadratischen Profil;
- Figur 9: eine Draufsicht auf die Vorrichtung nach den Figuren 1 bis 4 bei Aufnahme einer Stange mit einem Achtkantprofil;
- Figur 10: eine Draufsicht auf die Vorrichtung nach den Figuren 1 bis 4 bei Aufnahme einer Stange mit einem Sechskantprofil;
- Figur 11: eine schematische Darstellung einer Vorrichtung zum Befestigen eines Zubehörteils an einem Operationstisch gemäß einer weiteren Ausführungsform und
- Figur 12: eine schematische Darstellung einer Vorrichtung zum Befestigen eines Zubehörteils an einem Operationstisch gemäß einer weiteren Ausführungsform.

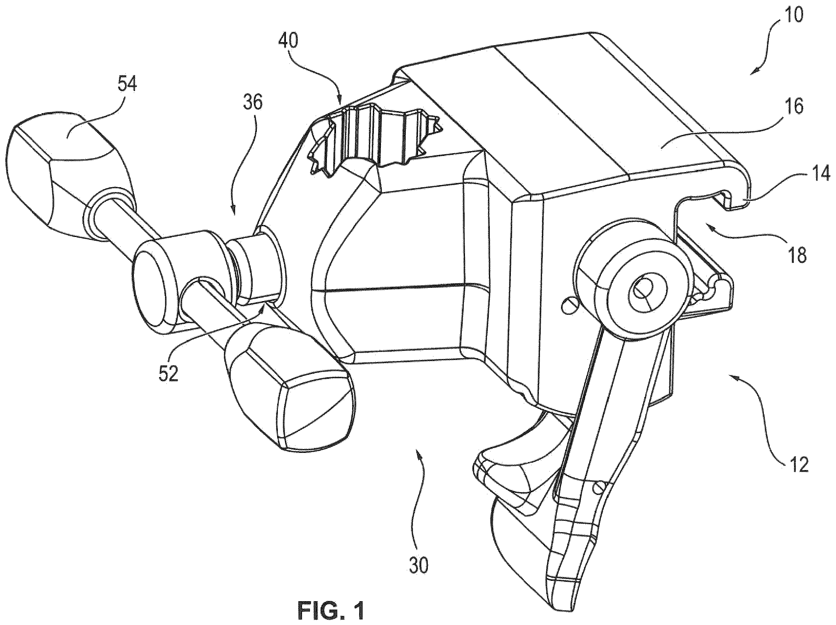

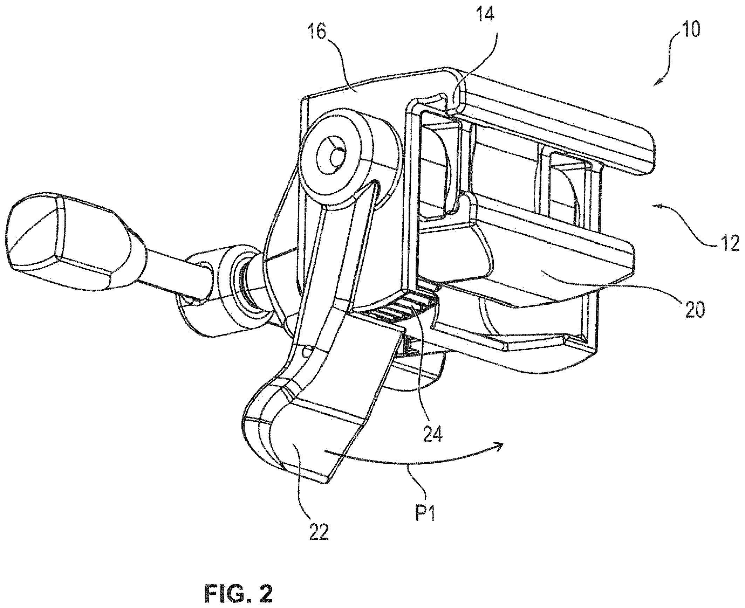

In den Figuren 1 und 2 ist jeweils eine schematische, perspektivische Darstellung einer ersten Ausführungsform einer Vorrichtung 10 zum Befestigen von Zubehörteilen an Schienen eines Operationstisches gezeigt. Die Vorrichtung 10 wird häufig auch als Kloben bezeichnet.

Die Vorrichtung 10 weist eine erste Befestigungseinheit 12 zur Befestigung der Vorrichtung 10 an den Schienen des Operationstisches auf. Diese erste Befestigungseinheit 12 umfasst einen ersten Haken 14, der als Teil eines Grundkörpers 16 der Vorrichtung 10 ausgebildet ist. Dieser Haken 14 kann über die obere Seite der Schiene des Operationstisches gehängt werden, so dass die Schiene in der Aussparung 18 aufgenommen ist.

Ferner umfasst die erste Befestigungseinheit 12 einen weiteren Haken 20, der über einen Hebel 22 manuell verstellt werden kann. Sobald die Vorrichtung 10 von oben auf die Schiene aufgesetzt wurde, wird der Hebel 22 aus der in Figur 2 gezeigten Position in Richtung des Pfeiles P1 bewegt, wodurch auch der zweite Haken 20 in Richtung des Pfeiles P1 bewegt wird, so dass er die Schiene des Operationstisches von unten umgreift und somit eine Klemmverbindung herstellt. Hierdurch wird eine sichere Befestigung auf einer Operationstischschiene erreicht. Insbesondere wird auch ein axiales Verschieben auf der Schiene vermieden. Der Hebel 22 umfasst ein in Figur 2 nicht sichtbares Rastelement, das in eine komplementäre Rastung 24 einrasten kann, so dass der Hebel 22 und somit der zweite Haken 20 in der gewünschten Position verbleiben.

An der der ersten Befestigungseinheit 12 abgewandten Seite des Grundkörpers 16 ist eine zweite Befestigungseinheit 30 zur Befestigung einer Stange eines Zubehörteils an der Vorrichtung 10 und somit an dem Operationstisch vorgesehen. Bei dem Zubehörteil kann es sich beispielsweise um eine Körperstütze, eine Armstütze und/oder einen Anästhesiebogen handeln. Diesen Zubehörteilen ist es gemein, dass sie eine Stange umfassen, die über die Vorrichtung 10 vertikal verlaufend am Operationstisch höhenverstellbar befestigt werden soll. Dies wird durch die wie im Folgenden näher beschrieben für eine Vielzahl von verschiedenen vorbestimmten Profilen der Stange ermöglicht.

Die zweite Befestigungseinheit 30 weist ein Durchgangsloch 40 auf, durch das die an der Vorrichtung 10 zu befestigende Stangen der Zubehörteile geführt werden. Das Loch 40 ist insbesondere derart ausgebildet, dass durch es sowohl Stangen mit einem quadratischen Profil, Stangen mit einem rechteckigen (nicht-quadratischen) Profil, als auch Stangen mit einem runden Profil mit oder ohne plane Fläche, Stangen mit einem Sechskantprofil als auch Stangen mit einem Achtkantprofil jeweils zwischen einem Mindest- und einem Höchstmaß geführt werden können.

Hierzu weist das Loch 40 insbesondere an der der Knebelschraube 36 zugewandten Seite eine ebene Fläche 42 als Seitenwandung auf, in der mittig eine V-förmige Nut 44 vorgesehen ist.
Die zweite Befestigungseinheit 30 weist ein Loch 52 auf, das bis in das Loch 40 hineinragt. In dem Loch 52 ist ein Gewinde vorgesehen, in das die Spannschraube 36 eingreift. Durch Drehen des Hebels 54 der Spannschraube 36 kann diese in axialer Richtung des Lochs 52 in das Loch 40 hinein und aus diesem heraus bewegt werden, wodurch die aufgenommenen Stangen, wie später noch genauer beschrieben wird, eingeklemmt werden.

Das Loch 40 ist derart ausgebildet, dass seine Längsachse bei planmäßiger Montage der Vorrichtung 10 an einer horizontal verlaufenden Schiene des Operationstisches vertikal ausgerichtet ist.

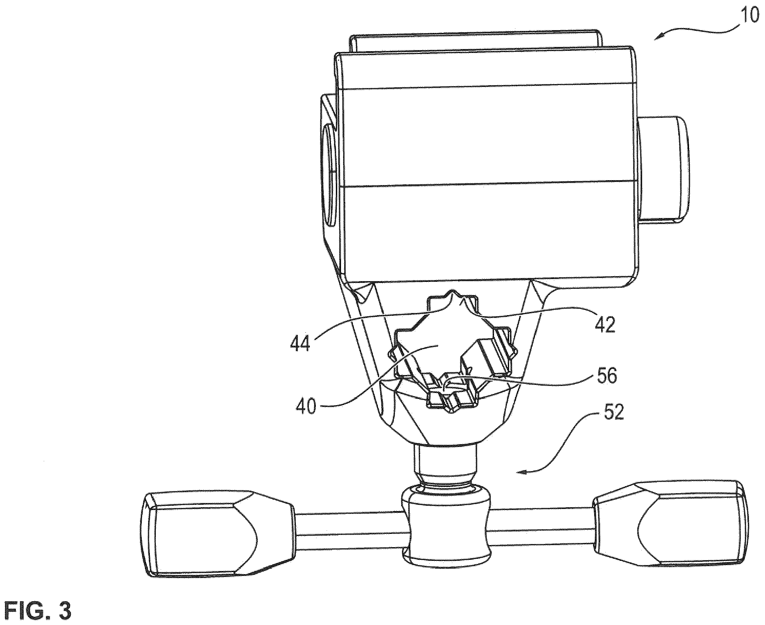

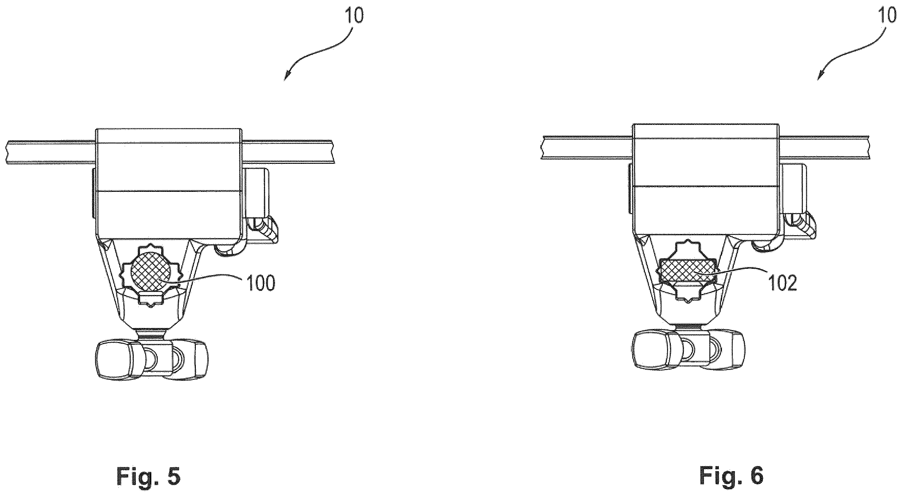

In Figur 3 ist eine weitere schematische perspektivische Darstellung der Vorrichtung 10 gezeigt, wobei hierbei der Blick insbesondere in das Loch 40 hineingerichtet ist. Das Loch 40 ist hierbei derart geformt, dass sowohl Stangen mit einem runden Profil, Stangen mit einem rechteckigen, nicht-quadratischen Profil, Stangen mit einem quadratischen Profil, Stangen mit einem Sechskantprofil als auch Stangen mit einem Achtkantprofil sicher innerhalb des Loches eingeklemmt werden können. Hierzu ist der Querschnitt des Loches 40 derart gewählt, dass alle zuvor genannten Profilarten an der entgegengesetzten Seite des Loches 40 an der Wandung des Loches entweder flächig oder zumindest entlang zweier Kontaktlinien anliegen, so dass sowohl ein Verrutschen, als auch ein Verdrehen sicher vermieden wird. Die entsprechende Kontaktierung kann insbesondere den Figuren 5 bis 10 für die verschiedenen zuvor genannten Profilarten gut entnommen werden.

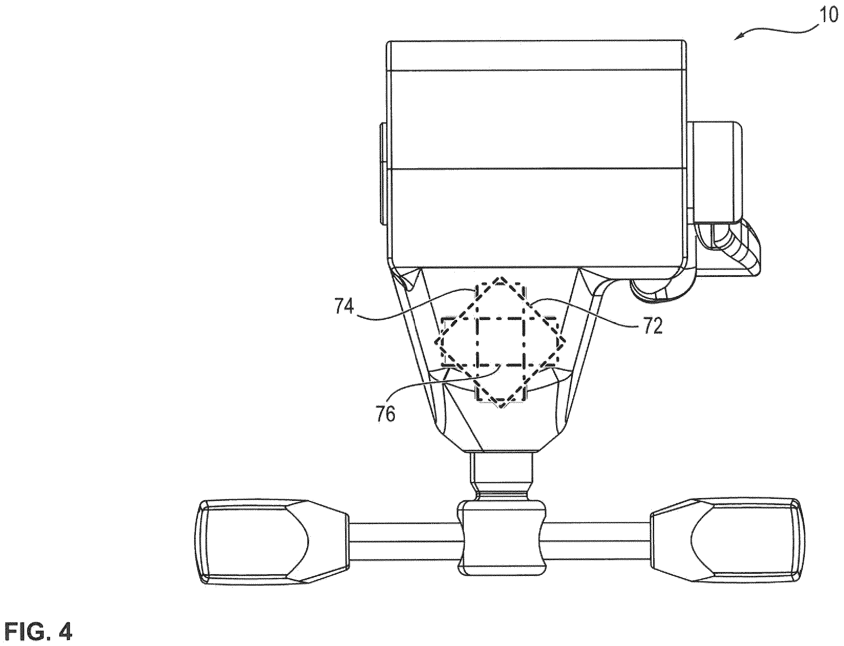

In Figur 4 ist eine weitere schematische perspektivische Darstellung der Vorrichtung 10 nach den Figuren 1 bis 3 gezeigt, wobei zur Erläuterung der Formgebung des Loches 40 über Strichlinien bzw. strichpunktierte Linien geometrische Formen in dem Loch 40 eingezeichnet sind.

Der Querschnitt des Loches 40 ergibt sich aus der Überlagerung eines Quadrates 72 sowie zweier Rechtecke 74, 76. Die Rechtecke sind identisch geformt und zueinander um 90° verdreht angeordnet. Das Quadrat 72 ist relativ zu den beiden Rechtecken um 45° gedreht angeordnet, wobei der Mittelpunkt von dem Quadrat 72 und die Mittelpunkte der Rechtecke 74, 76 in einem Punkt zusammenfallen. Die Diagonalen des Quadrates 72 sind länger als die langen Seiten der Rechtecke 74, 76, wodurch erreicht wird, dass das Quadrat an den kurzen Seiten der Rechtecke über diese übersteht, so dass sie V-förmige Nuten in der Kontur des Loches 40 ergeben. Insbesondere wird insgesamt betrachtet die Kontur des Loches 40 an manchen Teilen durch das Quadrat 72, an manchen Teilen durch das Rechteck 74 und an manchen Teilen durch das Rechteck 76 gebildet.

Wie im Folgenden im Zusammenhang mit den Figuren 5 bis 10 näher erläutert wird, können über diese spezielle Formgebung des Loches 40 alle gängigen Profilarten sicher über die Vorrichtung 10 eingespannt und somit an dem Operationstisch befestigt werden.

In Figur 5 ist die Aufnahme eines Stabes 100 mit einem runden Profil gezeigt. Dieser Stab liegt an der der Spannschraube 36 entgegengesetzten Seite über zwei Kontaktlinien an der Wandung des Loches 40 an und wird somit sicher fixiert.

Figur 6 zeigt eine Draufsicht auf die Vorrichtung 10 nach den Figuren 1 bis 4, wobei eine Stange 102 mit einem rechteckigen Profil eingespannt ist. Die Stange 102 liegt flächig an den durch das Rechteck 76 gebildeten Teilbereich des Umfangs des Loches 40 an, so dass auch die Stange 102 sicher fixiert werden kann.

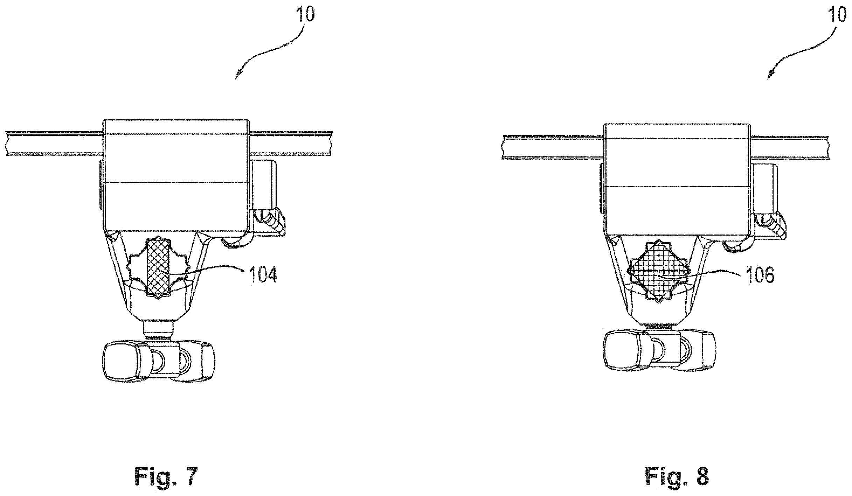

Figur 7 zeigt eine Draufsicht auf die Vorrichtung 10 nach den Figuren 1 bis 4, wobei eine Stange 104 mit einem rechteckigen Profil aufgenommen ist. Verglichen mit der Stange 102 in Figur 6 ist diese Stange 104 jedoch um 90° gedreht angeordnet. Diese Stange wird über die Spannschraube 36 an die durch das Rechteck 74 gebildeten Flächen gedrückt, so dass auch die Stange 104 rutsch- und drehsicher eingespannt werden kann.

In Figur 8 ist eine Draufsicht auf die Vorrichtung 10 gezeigt, wobei eine Stange 106 mit einem quadratischen Profil in dem Loch 40 aufgenommen ist. Hierbei sind die Kanten des quadratischen Profils in den über das Quadrat 72 gebildeten V-förmigen Nuten aufgenommen, so dass ein sicheres Halten gewährleistet wird.

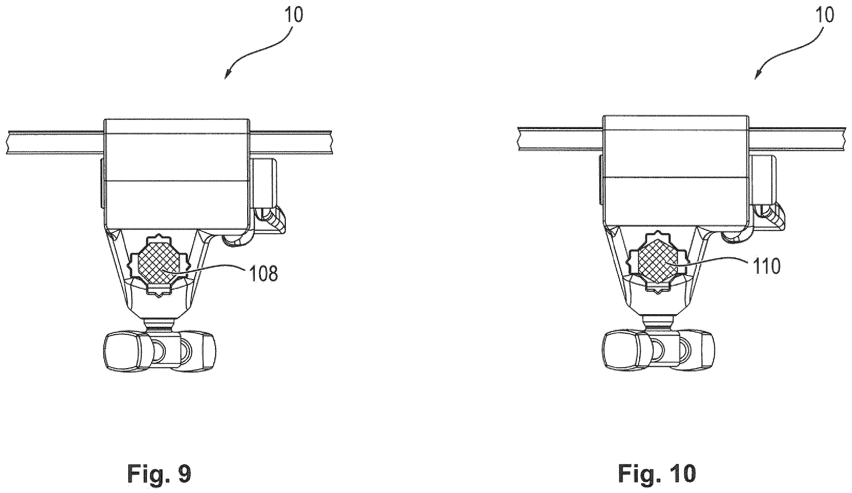

In Figur 9 ist eine weitere Draufsicht auf die Vorrichtung 10 gezeigt, wobei eine Stange 108 mit einem Achtkantprofil aufgenommen ist.

Figur 10 zeigt eine Draufsicht auf die Vorrichtung 10 nach den Figuren 1 bis 4, wobei eine Stange 110 mit einem Sechskantprofil 104 in dem Loch aufgenommen und sicher eingespannt ist.

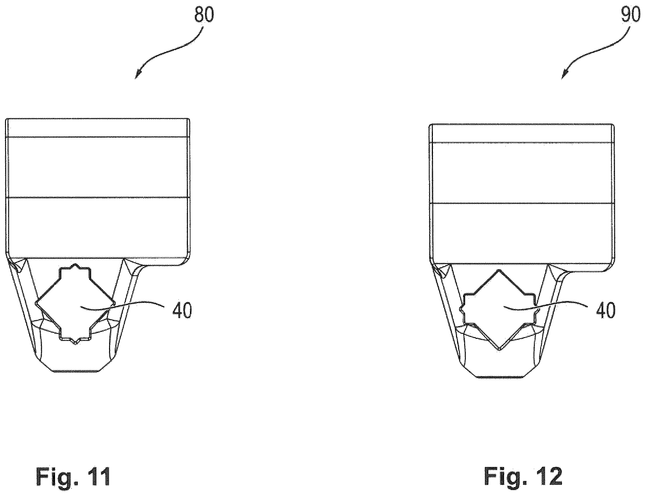

In Figur 11 ist eine schematische Darstellung einer Vorrichtung 80 zum Befestigen eines Zubehörteils an einem Operationstisch gemäß einer zweiten Ausführungsform gezeigt. Bei dieser zweiten Ausführungsform ist das Loch 40 anders geformt, nämlich es besteht nur aus der Überlagerung des Quadrates 72 und des Rechteckes 74.

Auch bei dieser Formgebung können nach wie vor eine Vielzahl unterschiedlicher Profilarten, jedoch nicht so viele Profilarten wie bei der ersten Ausführungsform aufgenommen werden. Insbesondere können keine Rechteckprofile aufgenommen werden, die entsprechend der Stange 102 in Figur 6 angeordnet sind.

In Figur 12 ist eine Draufsicht auf eine Vorrichtung 90 zur Befestigung eines Zubehörteils an einem Operationstisch gemäß einer dritten Ausführungsform gezeigt. Bei dieser dritten Ausführungsform ist das Loch 40 derart geformt, dass sein Querschnitt sich aus der Überlagerung des Quadrates 72 und des Rechtecks 76 ergibt. Bei dieser Ausführungsform können keine Stangen aufgenommen werden, die entsprechend der Stange 104 gemäß Figur 7 angeordnet sind.

Insgesamt wird durch die zuvor beschriebene Vorrichtung 10 erreicht, dass Stangen 100 bis 108 mit vielen verschiedenen vorbestimmten Profilen über die Vorrichtung 10 an dem Operationstisch befestigt werden können. Ferner wird jedenfalls vermieden, dass falsche Stangen im jeweils eingestellten Betriebszustand eingeführt werden, wodurch eine sichere Befestigung gewährleistet wird.

Somit benötigt der Anwender nur noch eine Vorrichtung 10, die für eine große Anzahl von Zubehörteilen verwendet wird. Darüber hinaus ist die Vorrichtung 10 nicht dauerhaft über eine stoffschlüssige Verbindung an den jeweiligen Zubehörteilen befestigt.

Eventuell kann die Vorrichtung 10 sogar am Operationstisch verbleiben, wenn der Patient umgebettet wird, da die Vorrichtung 10 nicht über den Operationstisch hinausragt und somit das Umbetten nicht behindert.

Darüber hinaus wird durch die Vorrichtung 10 insgesamt die Handhabung vereinfacht, da alle Zubehörteile durch die gleichen Handgriffe fixiert werden können und sich der Anwender nicht jeweils auf individuelle Befestigungsvorrichtungen einstellen muss. Darüber hinaus wird anders als bei Radialstellkolben ein Wegkippen der Vorrichtung 10, wenn die Befestigung gelöst ist, vermieden.

Ferner ist es vorteilhaft, dass die quadratischen Profile an der Kante geklemmt werden, wodurch, anders als beim flächigen Klemmen, bei hohlen Profilen eine Verformung vermieden wird.

### Bezugszeichenliste

- 10, 80, 90: Vorrichtung
- 12: erste Befestigungseinheit
- 14: Haken
- 16: Grundkörper
- 18: Aussparung
- 20: Haken
- 22: Hebel
- 24: Rastung
- 30: zweite Befestigungseinheit
- 36: Spannschraube
- 40: Loch
- 42: Fläche
- 44: V-förmige Nut
- 52: Loch
- 54: Hebel
- 56: Kontaktfläche
- 72: Quadrat
- 74, 76: Rechteck
- 100 bis 110: Stange
- P1: Richtung

## Patentansprüche

1. Vorrichtung zum Befestigen eines Zubehörteils an einem Operationstisch
mit einer ersten Befestigungseinheit (12) zur Befestigung der Vorrichtung (10, 80, 90) an dem Operationstisch, insbesondere an einer Schiene des Operationstischs, und
mit einer zweiten Befestigungseinheit (30) zur Befestigung einer Stange (100 bis 110) des Zubehörteils an der Vorrichtung (10, 80, 90),
wobei die zweite Befestigungseinheit (30) ein Loch (40) zur Aufnahme einer Stange (100 bis 110) des Zubehörteils umfasst,
die zweite Befestigungseinheit (30) eine Klemmeinheit (36) zum Einklemmen einer im Loch (40) aufgenommenen Stange (100 bis 110) umfasst, und
der Querschnitt des Loches (40) derart ausgebildet ist, dass in dem Loch sowohl Stangen (100 bis 110) mit einer ersten Profilart als auch Stangen (100 bis 110) mit mindestens einer zweiten Profilart mit Hilfe der Klemmeinheit (36), insbesondere formschlüssig, befestigbar sind,
**dadurch gekennzeichnet, dass** sich der Querschnitt des Loches (40) aus der Überlagerung eines Quadrates (72) und eines oder zweier Rechtecke (74, 76) ergibt, und dass die Diagonale des Quadrates (72) länger ist als die längere Seite eines jeden Rechtecks (74, 76).

2. Vorrichtung (10, 80, 90) nach Anspruch 1, **dadurch gekennzeichnet, dass** der Querschnitt des Loches (40) derart ausgebildet ist, dass in dem Loch (40) sowohl Stangen (100 bis 110) mindestens einer dritten Profilart befestigbar sind.

3. Vorrichtung (10, 80, 90) nach Anspruch 1 oder 2, **dadurch gekennzeichnet, dass** der Querschnitt des Loches (40) derart ausgebildet ist, dass in dem Loch (40) Stangen (100 bis 110) mit einem kreisrunden Profil, Stangen (100 bis 110) mit einem kreisrunden Profil mit planen Flächen, Stangen (100 bis 110) mit einem rechteckigen Profil, Stangen (100 bis 110) mit einem quadratischen Profil und/oder Stangen (100 bis 110) mit einem n-eckigen, achsensymmetrischen Profil befestigbar sind.

4. Vorrichtung (10, 80, 90) nach einem der vorhergehenden Ansprüche, **dadurch gekennzeichnet, dass** der Querschnitt des Loches (40) derart ausgebildet ist, dass sowohl Stangen (100 bis 110) der ersten Profilart als auch Stangen (100 bis 110) der zweiten Profilart das Loch (40) an der der Klemmeinheit (36) entgegensetzten Seite des Loches (40) an mindestens drei punktförmigen Bereichen kontaktieren.

5. Vorrichtung (10, 80, 90) nach einem der vorhergehenden Ansprüche, **dadurch gekennzeichnet, dass** der Querschnitt des Loches (40) derart ausgebildet ist, dass sowohl Stangen (100 bis 110) der ersten Profilart als auch Stangen (100 bis 110) der zweiten Profilart das Loch (40) an der der Klemmeinheit (36) entgegensetzten Seite des Loches (40) entlang mindestens zweier Linien kontaktieren.

6. Vorrichtung (10, 80, 90) nach einem der vorhergehenden Ansprüche, **dadurch gekennzeichnet, dass** der Querschnitt des Loches (40) derart ausgebildet ist, dass zumindest Stangen (100 bis 110) der ersten Profilart das Loch (40) an der der Klemmeinheit (36) entgegensetzten Seite des Loches (40) flächig kontaktieren.

7. Vorrichtung (10, 80, 90) nach einem der vorhergehenden Ansprüche, **dadurch gekennzeichnet, dass** der Querschnitt des Loches (40) derart ausgebildet ist, dass sowohl Stangen (100 bis 110) der ersten Profilart als auch Stangen (100 bis 110) der zweiten Profilart das Loch (40) an der der Klemmeinheit (36) entgegensetzten Seite des Loches (40) flächig kontaktieren.

8. Vorrichtung (10, 80, 90) nach einem der vorhergehenden Ansprüche, **dadurch gekennzeichnet, dass** der Querschnitt des Loches (40) derart ausgebildet ist, dass sowohl bei Stangen (100 bis 110) der ersten Profilart als auch bei Stangen (100 bis 110) der zweiten Profilart ein seitliches Verrutschen der entsprechend aufgenommen Stange (100 bis 110) über den Kontakt zur Wandung des Loches (40) vermieden wird.

9. Vorrichtung (10, 80, 90) nach einem der vorhergehenden Ansprüche, **dadurch gekennzeichnet, dass** das Quadrat (72) relativ zu jedem Rechteck (74, 76) um 45° gedreht ist.

10. Vorrichtung (10, 80, 90) nach einem der vorhergehenden Ansprüche, **dadurch gekennzeichnet, dass** das Quadrat (72) und jedes Rechteck (74, 76) übereinander überstehen, so dass der Querschnitt des Loches (40) teilweise von dem Quadrat (72) und teilweise von dem Rechteck (74, 76) begrenzt ist.

11. Vorrichtung (10, 80, 90) nach einem der vorhergehenden Ansprüche, **dadurch gekennzeichnet, dass** sich der Querschnitt des Loches (40) aus der Überlagerung eines Quadrates (72) und zweier Rechtecke (74, 76) ergibt, dass die Rechtecke (74,76) um 90° zueinander gedreht sind, und dass das Quadrat (72) relativ zu den Rechtecken (74, 76) jeweils um 45° gedreht ist.

12. Vorrichtung (10, 80, 90) nach einem der vorhergehenden Ansprüche, **dadurch gekennzeichnet, dass** sich der Querschnitt des Loches (40) aus der Überlagerung eines Quadrates (72) und zweier Rechtecke (74, 76) ergibt, und dass das Quadrat (72) und die Rechtecke (74, 76) jeweils übereinander überstehen, so dass der Querschnitt des Loches (40) teilweise von dem Quadrat (72), teilweise von dem einem Rechteck (74) und teilweise von dem anderen Rechteck (76) begrenzt ist.

13. Vorrichtung (10, 80, 90) nach einem der vorhergehenden Ansprüche, **dadurch gekennzeichnet, dass** sich der Querschnitt des Loches (40) aus der Überlagerung eines Quadrates (72) und zweier Rechtecke (74, 76) ergibt, und dass die Rechtecke (74, 76) identisch geformt sind.

14. Vorrichtung (10, 80, 90) nach einem der vorhergehenden Ansprüche, **dadurch gekennzeichnet, dass** die Klemmeinheit (36) eine Spannschraube, insbesondere eine Knebelschraube, umfasst, wobei vorzugsweise die Spannschraube (36) eine plane Kontaktfläche (56) zum Kontaktieren der einzuklemmenden Stangen (100 bis 110) umfasst.

## Claims

1. A device for securing an accessory to an operating table,
comprising a first securing unit (12) for securing the device (10, 80, 90) to the operating table, in particular to a rail of the operating table, and
comprising a second securing unit (30) for securing a rod (100 to 110) of the accessory to the device (10, 80, 90),
wherein the second securing unit (30) comprises an opening (40) for receiving a rod (100 to 110) of the accessory,
the second securing unit (30) comprises a clamping unit (36) for clamping a rod (100 to 110) received in the opening (40), and
the cross section of the opening (40) is configured such that both rods (100 to 110) having a first profile type and rods (100 to 110) having at least a second profile type can be secured in the opening, in particular in a positively locked connection, with the aid of the clamping unit (36), **characterized in that** the cross section of the opening (40) results from the superimposition of a square (72) and one or two rectangles (74, 76), and **in that** the diagonal of the square (72) is longer than the longer side of each rectangle (74, 76).

2. The device (10, 80, 90) according to claim 1, **characterized in that** the cross section of the opening (40) is configured such that rods (100 to 110) of at least a third profile type can also be secured in the opening (40).

3. The device (10, 80, 90) according to claim 1 or 2, **characterized in that** the cross section of the opening (40) is configured such that rods (100 to 110) having a circular profile, rods (100 to 110) having a circular profile with flat surfaces, rods (100 to 110) having a rectangular profile, rods (100 to 110) having a square profile, and/or rods (100 to 110) having an n-gonal, axis-symmetrical profile can be secured in the opening (40).

4. The device (10, 80, 90) according to any one of the preceding claims, **characterized in that** the cross section of the opening (40) is configured such that both rods (100 to 110) of the first profile type and rods (100 to 110) of the second profile type contact the opening (40) in at least three point-contact regions on the side of the opening (40) opposite the clamping unit (36).

5. The device (10, 80, 90) according to any one of the preceding claims, **characterized in that** the cross section of the opening (40) is configured such that both rods (100 to 110) of the first profile type and rods (100 to 110) of the second profile type contact the opening (40) along at least two lines on the side of the opening (40) opposite the clamping unit (36) .

6. The device (10, 80, 90) according to any one of the preceding claims, **characterized in that** the cross section of the opening (40) is configured such that at least rods (100 to 110) of the first profile type contact the opening (40) in a flat contact on the side of the opening (40) opposite the clamping unit (36).

7. The device (10, 80, 90) according to any one of the preceding claims, **characterized in that** the cross section of the opening (40) is configured such that both rods (100 to 110) of the first profile type and rods (100 to 110) of the second profile type contact the opening (40) in a flat contact on the side of the opening (40) opposite the clamping unit (36).

8. The device (10, 80, 90) according to any one of the preceding claims, **characterized in that** the cross section of the opening (40) is configured such that, both with rods (100 to 110) of the first profile type and with rods (100 to 110) of the second profile type, lateral slipping of the correspondingly received rod (100 to 110) is prevented via the contact with the wall of the opening (40).

9. The device (10, 80, 90) according to any one of the preceding claims, **characterized in that** the square (72) is rotated by 45° relative to each rectangle (74, 76).

10. The device (10, 80, 90) according to any one of the preceding claims, **characterized in that** the square (72) and each rectangle (74, 76) protrude beyond one another, so that the cross section of the opening (40) is delimited partially by the square (72) and partially by the rectangle (74, 76).

11. The device (10, 80, 90) according to any one of the preceding claims, **characterized in that** the cross section of the opening (40) results from the superimposition of one square (72) and two rectangles (74, 76), **in that** the rectangles (74, 76) are rotated by 90° relative to one another, and **in that** the square (72) is rotated by 45° relative to each of the rectangles (74, 76).

12. The device (10, 80, 90) according to any one of the preceding claims, **characterized in that** the cross section of the opening (40) results from the superimposition of one square (72) and two rectangles (74, 76), and **in that** the square (72) and the rectangles (74, 76) each protrude beyond one another, so that the cross section of the opening (40) is delimited partially by the square (72), partially by the one rectangle (74), and partially by the other rectangle (76).

13. The device (10, 80, 90) according to any one of the preceding claims, **characterized in that** the cross section of the opening (40) results from the superimposition of one square (72) and two rectangles (74, 76), and **in that** the rectangles (74, 76) are identical in shape.

14. The device (10, 80, 90) according to any one of the preceding claims, **characterized in that** the clamping unit (36) comprises a tensioning screw, in particular a tommy screw, wherein the tensioning screw (36) preferably has a flat contact surface (56) for contacting the rods (100 to 110) to be clamped.

## Revendications

1. Dispositif pour fixer un accessoire à une table d'opération
comportant une première unité de fixation (12) pour fixer le dispositif (10, 80, 90) à la table d'opération, en particulier à un rail de la table d'opération, et
comportant une seconde unité de fixation (30) pour fixer une tige (100 à 110) de l'accessoire au dispositif (10, 80, 90),
dans lequel la seconde unité de fixation (30) comprend un trou (40) pour recevoir une tige (100 à 110) de l'accessoire,
la seconde unité de fixation (30) comprend une unité de serrage (36) pour serrer une tige (100 à 110) reçue dans le trou (40), et
la section transversale du trou (40) est conçue de sorte que des tiges (100 à 110) présentant un premier type de profil aussi bien que des tiges (100 à 110) présentant au moins un second type de profil peuvent être fixées dans le trou à l'aide de l'unité de serrage (36), en particulier par complémentarité de forme,
**caractérisé en ce que** la section transversale du trou (40) résulte de la superposition d'un carré (72) et d'un ou de deux rectangles (74, 76), et **en ce que** la diagonale du carré (72) est plus longue que le côté le plus long de chaque rectangle (74, 76).

2. Dispositif (10, 80, 90) selon la revendication 1, **caractérisé en ce que** la section transversale du trou (40) est conçue de sorte que des tiges (100 à 110) d'au moins un troisième type de profil peuvent également être fixées dans le trou (40) .

3. Dispositif (10, 80, 90) selon la revendication 1 ou 2, **caractérisé en ce que** la section transversale du trou (40) est conçue de sorte que des tiges (100 à 110) présentant un profil circulaire, des tiges (100 à 110) présentant un profil circulaire à surfaces planes, des tiges (100 à 110) présentant un profil rectangulaire, des tiges (100 à 110) présentant un profil carré et/ou des tiges (100 à 110) présentant un profil axisymétrique à n angles peuvent être fixées dans le trou (40) .

4. Dispositif (10, 80, 90) selon l'une des revendications précédentes, **caractérisé en ce que** la section transversale du trou (40) est conçue de sorte que des tiges (100 à 110) du premier type de profil aussi bien que des tiges (100 et 110) du second type de profil entrent en contact avec le trou (40) sur le côté du trou (40) opposé à l'unité de serrage (36) dans au moins trois zones en forme de points.

5. Dispositif (10, 80, 90) selon l'une des revendications précédentes, **caractérisé en ce que** la section transversale du trou (40) est conçue de sorte que des tiges (100 à 110) du premier type de profil aussi bien que des tiges (100 et 110) du second type de profil entrent en contact avec le trou (40) sur le côté du trou (40) opposé à l'unité de serrage (36) le long d'au moins deux lignes.

6. Dispositif (10, 80, 90) selon l'une des revendications précédentes, **caractérisé en ce que** la section transversale du trou (40) est conçue de sorte qu'au moins des tiges (100 à 110) du premier type de profil entrent en contact face contre face avec le trou (40) sur le côté du trou (40) opposé à l'unité de serrage (36).

7. Dispositif (10, 80, 90) selon l'une des revendications précédentes, **caractérisé en ce que** la section transversale du trou (40) est conçue de sorte que les tiges (100 à 110) du premier type de profil aussi bien que les tiges (100 et 110) du second type de profil entrent en contact face contre face avec le trou (40) sur le côté du trou (40) opposé à l'unité de serrage (36).

8. Dispositif (10, 80, 90) selon l'une des revendications précédentes, **caractérisé en ce que** la section transversale du trou (40) est conçue de sorte qu'un glissement latéral de la tige reçue correspondante (100 à 110) est évité lors du contact avec la paroi du trou (40) aussi bien pour les tiges (100 à 110) du premier type de profil que pour les tiges (100 et 110) du second type de profil.

9. Dispositif (10, 80, 90) selon l'une des revendications précédentes, **caractérisé en ce que** le carré (72) est tourné de 45° par rapport à chaque rectangle (74, 76).

10. Dispositif (10, 80, 90) selon l'une des revendications précédentes, **caractérisé en ce que** le carré (72) et chaque rectangle (74, 76) font saillie l'un au-dessus de l'autre, de sorte que la section transversale du trou (40) est délimitée partiellement par le carré (72) et partiellement par le rectangle (74, 76).

11. Dispositif (10, 80, 90) selon l'une des revendications précédentes, **caractérisé en ce que** la section transversale du trou (40) résulte de la superposition d'un carré (72) et de deux rectangles (74, 76), **en ce que** les rectangles (74, 76) sont tournés de 90° les uns par rapport aux autres, et **en ce que** le carré (72) est tourné de 45° par rapport aux rectangles (74, 76).

12. Dispositif (10, 80, 90) selon l'une des revendications précédentes, **caractérisé en ce que** la section transversale du trou (40) résulte de la superposition d'un carré (72) et de deux rectangles (74, 76), et **en ce que** le carré (72) et les rectangles (74, 76) font saillie les uns sur les autres de sorte que la section transversale du trou (40) est délimitée partiellement par le carré (72), partiellement par l'un des rectangles (74) et partiellement par l'autre des rectangles (76) .

13. Dispositif (10, 80, 90) selon l'une des revendications précédentes, **caractérisé en ce que** la section transversale du trou (40) résulte de la superposition d'un carré (72) et de deux rectangles (74, 76), et **en ce que** les rectangles (74, 76) sont formés de manière identique.

14. Dispositif (10, 80, 90) selon l'une des revendications précédentes, **caractérisé en ce que** l'unité de serrage (36) comprend une vis de serrage, en particulier une vis à oreilles, dans lequel, de préférence, la vis de serrage (36) présente une surface de contact plane (56) pour entrer en contact avec les tiges à serrer (100 à 110).
